(19) Europäisches Patentamt

European Patent Office

Office européen des brevets

(11) Veröffentlichungsnummer : **0 004 668**
**B1**

(12) **EUROPÄISCHE PATENTSCHRIFT**

(45) Veröffentlichungstag der Patentschrift :
17.06.81

(51) Int. Cl.³ : **C 07 G  7/00, A 61 K 35/16,**
**A 61 K 35/407**

(21) Anmeldenummer : 79101041.6

(22) Anmeldetag : 05.04.79

(54) Faktor zur Stimulation der Proliferationsrate von Leberzellen und diesen Faktor als Wirkstoff enthaltende Arzneimittel.

(30) Priorität : 07.04.78 DE 2814981

(43) Veröffentlichungstag der Anmeldung :
17.10.79 (Patentblatt 79/21)

(45) Bekanntmachung des Hinweises auf die Patenterteilung : 17.06.81 Patentblatt 81/24

(84) Benannte Vertragsstaaten :
CH FR GB IT NL SE

(56) Entgegenhaltungen :
CHEMICAL ABSTRACTS,
Band 78, Nr. 19, 1973 (Columbus, Ohio, USA)
C.D. JACKSON et al. « Effects of 2-acetylamino-
fluorene on liver cell proliferation after partial
hepatectomy of female rats »
Seite 103, linke Spalte, Abstract Nr. 120 023s

CHEMICAL ABSTRACTS,
Band 83, Nr. 23, 1975 (Columbus, Ohio, USA)
T.I. VALYAKINA et al. « Soluble and membranebound neuraminidase in regenerating rat liver »
Seite 256, rechte Spalte, Abstract Nr. 197076j

(73) Patentinhaber : Max-Planck-Gesellschaft zur Förderung der Wissenschaften e.V.
Bunsenstrasse 10
D-3400 Göttingen (DE)

(72) Erfinder : Ruhenstroth-Bauer, Gerhard, Prof. Dr.
Spitzelbergerstrasse 11
D-8032 Gräfelfing (DE)
Erfinder : Goldberg, Michel
Hohenzollernplatz 8
D-8000 München 40 (DE)
Erfinder : Silz, Siegfried, Dr.
Innere Wiener Strasse 26/IV
D-8000 München 80 (DE)
Erfinder : Strecker, Wolfgang
Allingerstrasse 21
D-8031 Eichenau (DE)

(74) Vertreter : Dreiss, Uwe, Dr. jur.,Dipl.-Ing., M.Sc. et al
Dreiss & Fuhlendorf Schikstrasse 2
D-7000 Stuttgart 1 (DE)

## Faktor zur Stimulation der Proliferationsrate von Leberzellen, und diesen Faktor als Wirkstoff enthaltende Arzneimittel

Die Erfindung betrifft einen Faktor zur Stimulation der Proliferationsrate von Leberzellen, und die diesen Faktor als Wirkstoff enthaltenden Arzneimittel.

Es ist bekannt, daß das Leberwachstum durch zwei Regelkreise beherrscht wird, von denen einer das Volumen der Leberzellen und der andere die Anzahl der Leberzellen regelt. Man weiß, daß bei einer operativen Entfernung eines Teils der Leber (Teilhepatektomie) eine gegenüber dem Normalzustand stark erhöhte Proliferationsrate der Leberzellen, also eine starke Erhöhung der Zellteilungsaktivität auftritt. Dies hat eine starke Erhöhung der durch die Operation verminderten Anzahl der Leberzellen zur Folge, bis die Ausgangszahl der Leberzellen etwa wieder erreicht ist. Außerdem ist eine solche Vermehrung der Anzahl der Leberzellen bei foetalen Säugern oder solchen in den ersten Wochen nach der Geburt zu beobachten. Diese Vermehrung erfolgt bis auf eine bestimmte Zahl. Von da ab erfolgt dann das weitere Leberwachstum durch Vergrößerung der Zellvolumina.

Man hat bereits vermutet, daß die Regelung der Proliferationsrate der Leberzellen durch einen Leberzellproliferationsfaktor erfolgt. Dabei hat man allerdings auch gleichermaßen erwogen, daß ein normalerweise die Zellteilung hemmender Faktor gegeben ist, der bei Teilhepatektomie durch bisher unbekannte Einflüsse wegfällt (vgl. z.B. die Zusammenfassung des derzeitigen Kenntnisstandes in *Bucher, N.L.R.* und *Malt, R.A.,* Regeneration of Liver and Kidney, Little, Brown & Co., Boston (1971), S. 245 ff).

Es ist Aufgabe der Erfindung, Faktoren zu gewinnen, die zu einer Erhöhung der Anzahl der Leberzellen durch erhöhte Zellteilungsaktivität, also zu einer Stimulation der Proliferationsrate der Leberzellen führt.

Diese Aufgabe wird durch die im Kennzeichen des Anspruchs 1 angegebenen Merkmale gelöst. Dabei wurde ferner ein Vorfaktor gefunden, der die genannte Aktivität in einer durch Einwirkung von Neuraminidase aktivierbaren Form enthält. Extrakte, die die Faktoren in konzentrierter und weitgehend entgifteter Form enthalten, werden angegeben, sowie ferner Verfahren zu deren Herstellung.

Auf diese Weise ist es erstmalig gelungen, einen den Leberzellproliferationsfaktor (im folgenden : Faktor) enthaltenden Wirkstoff durch Extraktion der Restlebern bzw. — als Vorform — durch Extraktion des Blutplasmas teilhepatektomierter Versuchstiere oder aus den Lebern bzw. Blutplasma foetaler Versuchstiere oder solcher in den ersten Wochen nach der Geburt mit einem einfachen Verfahren anzugeben. Es konnte geklärt werden, daß es sich bei dem Faktor um Proteine oder proteinhaltige Stoffe mit einem Molekulargewicht von ca. 30 000 bis 50 000 D handelt. Die unterste Grenze des Molekulargewichtes dürfte bei ca. 20 000 oder 25 000 liegen.

Damit sind sie eindeutig herstellbar und definierbar. Die angegebenen Herstellungsverfahren sind, wenngleich an sich äußerst vorteilhaft und einfach, lediglich beispielhaft. Es kann erwartet werden, daß nach Beschreibung des Leberzellenproliferationsfaktors in dieser Form in rascher Folge weitere Herstellungsmöglichkeiten, einschließlich einer Synthese, entwickelt bzw. gefunden werden.

Es besteht ein weiteres medizinisches Anwendungsfeld für einen Leberzellenproliferationsfaktor. Prinzipiell können damit alle Krankheiten behandelt werden, bei denen die Leberzellen in irgendeiner Weise krankhaft sind und das Wachstum neuer gesunder Leberzellen eine geeignete Therapie darstellt (Hepatitis, Leberzirrhose). Dabei ist von besonderem Vorteil, daß die gemäß der Erfindung gefundene Substanz zwar organspezifisch (d.h. also nur das Wachstum von Leberzellen, nicht etwa Nieren- oder Milzzellen anregend), jedoch nicht speziesspezifisch wirkt, so daß u.a. aus teilhepatektomierten Lebern einer bestimmten Spezies (Ratte) gewonnene Substanzen auch bei anderen Spezies wirksam sein werden ; für Mäuse konnte dies nachgewiesen werden.

Für diesen Faktor empfiehlt sich in Anlehnung an die übliche Bildung wissenschaftlicher Nomenklatur (z.B. Erythropoetin) der Name « Hepatopoetin ».

Im folgenden wird ein erster Weg zur Konzentrierung des Faktors aus Restlebern teilhepatektomierter Versuchstiere beschrieben :

Die Versuche wurden mit weiblichen 95-105 g schweren SPF-Wistar-Ratten (Institut für Strahlen- und Umweltforschung, Neuherberg/München) durchgeführt. Sie bekamen vor und nach der Behandlung als Nahrung Wasser und das unter dem Handelsnamen Altromin erhältliche Präparat in beliebiger Menge.

Bei sämtlichen Tieren erfolgte eine 68 %-ige Teilhepatektomie, d.h. eine Teilentfernung der Leber, und zwar im Hinblick auf den Tagesgang der Proliferationsaktivität der Leberzellen bei normalen Ratten in der Zeit von 19 bis 21 Uhr abends ; die Teilhepatektomie erfolgte nach *Higgins* u. *Anderson* (Arch. Path. (Chicago) 12, 186 (1931)).

Die Tötung der Tiere erfolgte jeweils 12 Stunden nach der Teilhepatektomie. Die Tiere wurden unter leichter Narkose entblutet und die Restlebern entfernt ; vor Entfernung wurden die Lebern von der Pfortader (V. portae) mit 0,9 % NaCl-Lösung durchspült. Die Restlebern von jeweils 3 Tieren wurden zusammen mit der 4-fachen (Gewichts-) Menge (w/w) zweifach destillierten Wassers (aqua bidest.) in einem Elvehjem-Potter homogenisiert. Dabei wurden die Leberzellen zerkleinert und möglichst vollständig zerstört. Das genannte Gerät ist bekannt ; die Leberzellen wurden in einem Glaszylinder zwischen diesem und einem darin rotierenden Teflonstift gebracht und durch die Scherkräfte geöffnet bzw. zerrieben. Auf diese Weise wurde sichergestellt, daß in den Zellen enthaltene Stoffe für die nachfolgende Konzentration bzw. Isolierung zugänglich werden.

Diese Leberzellhomogenate wurden mit einer Salzsäure (HCl-)Lösung in der Konzentration 0,1 N auf den pH-Wert 5,5 gebracht. Diese Ansäuerung ist ein wichtiges Selektionsmittel zur Ausscheidung einer großen Anzahl von Proteinen: sie werden auf diese Weise ausgefällt und damit dem weiteren Konzentrations- bzw. Isolationsprozeß entzogen. Anschließend wurden die Leberhomogenate bei einer Temperatur von 95 °C für die Dauer von 20 Minuten hitzedenaturiert. Dies hat den Zweck, weitere Bestandteile der Homogenate, und zwar die, die bei dieser Hitze und bei diesem pH-Wert nicht beständig sind, auszufällen.

Durch diese Verfahrensschritte, nämlich die Ansäuerung auf 5,5 pH und die Hitzedenaturierung wird also ein großer Anteil der Bestandteile der Leberzellen entfernt. Anschließend erfolgt eine Zentrifugierung für die Dauer von 15 Minuten mit 4 000 g (Minifuge Christ, Osterrode/Harz). Der Überstand nach der Zentrifugierung enthält somit nur diejenigen der insgesamt im ursprünglichen Homogenat enthaltenen Wirkstoffe, die bei pH = 5,5 und 95 °C stabil sind, diese jedoch in hoch konzentrierter Form. Um auch noch diejenigen Teile dieser Wirkstoffe zu erfassen, die in evtl. in den bei der Zentrifugierung enthaltenen Niederschlägen enthalten sind, wurden die Niederschläge wieder mit aqua bidest. auf das ursprüngliche Volumen aufgefüllt, erneut einer Ansäuerung auf pH = 5,5 und einer Hitzedenaturierung unterzogen und zentrifugiert. Dies wurde insgesamt zweimal wiederholt.

Die Überstände dieser nunmehr insgesamt drei Zentrifugationsvorgänge wurden zusammengegeben und lyophilisiert, d.h. unter Wasserentzug in Vakuum einer Gefriertrocknung unterzogen, so daß sie dann in pulverisierter Form zur Verfügung standen. Die Aufbewahrung bis zur Weiterverwendung erfolgte bei − 20 °C. Diese Substanz bildete den Extrakt der Restlebern der teilhepatektomierten Versuchstiere (*TL-Extrakt*).

Kontrolltiere mit nicht teilhepatektomierten Lebern wurden zur selben Zeit wie die Tiere mit teilhepatektomierten Lebern getötet. Ihnen wurde die vollständige Leber nach Durchspülung mit einer 0,9 %-igen NaCl-Lösung entnommen. Eine Menge Leber der Kontrolltiere, die der Menge der Restlebern der Versuchstiere äquivalent war, wurde ebenso wie die Restlebern der Versuchstiere behandelt, d.h. zunächst auf pH 5,5 gebracht bei 95 °C hitzedenaturiert und anschließend zentrifugiert, wobei die letzteren beiden Verfahrensschritte jeweils für die Niederschläge zweimal wiederholt wurden. Die Überstände wurden ebenfalls lyophilisiert und bildeten den Extrakt von Tieren mit normaler Leber (*NL-Extrakt*).

Die Überstände aus der Behandlung der Restlebern der Versuchstiere, d.h. der TL-Extrakte enthält den Leberzellprolieferationsfaktor Hepatopoetin. Dies wurde wie folgt nachgewiesen:

Der TL-Extrakt wurde in 0,9 %-NaCl-Lösung gelöst. Eine Menge von 2 ml der Lösung wurde normalen Ratten intraperitoneal (i.p.) injiziert. Den Kontrolltieren wurde dieselbe Menge des von den nicht teilhepatektomierten Normaltieren gewonnenen NL-Extraktes injiziert. Weiteren Kontrolltieren wurde eine 0,9 %-ige NaCl-Lösung i.p. injiziert.

Die Messung der Proliferation der Leberzellen nach Injektion des TL-Extraktes erfolgte nun durch Messung der DNA-Synthese. Diese kann dadurch efolgen, daß die Menge von spezifisch in die DNA eingebauten radioaktiven Substanzen gemessen wird, nämlich von $^3$H-Methylthymidin (spezielle Aktivität 25 Ci/mmol ; Radiochem. Center, Amersham).

Den Versuchstieren und den Kontrolltieren wurde 19 Stunden nach der Injektion der Extrakte TL bzw. NL 50 µCi $^3$H-Methylthymidin injiziert. Nach einer Stunde wurden die Tiere getötet. Die Leber wurde entfernt und bei − 20 °C aufbewahrt.

Darauf erfolgte eine Extraktion der DNA der Leber nach *Weinbren*, K. u. *Woodward*, E. (Br. J. exp. Path. 45, 442-449 (1964)). Ein Teil dieses Extraktes wurde zu einer Radioaktivitätsmessung verwendet. Dazu wurden 1,5 ml der PCA (Perchloressigsäure)-haltigen Lösung mit 0,5 ml NaOH in der Konzentration 1 N neutralisiert. Die entstehende Lösung wurde in einem Scintilationsgläschen mit 5 ml Triton X100 und 10 Ml Toluol (0,6 PPO ; PPO = 1,5-Diphenyloxazol) versetzt. Mit Hilfe eines Liquid-Scintilationszählers (Intertechnique, Paris) wurde dann die Radioaktivität als Anzahl der Zerfälle pro Minute (Zpm) ermittelt. Ein weiterer Teil des DNA-Extraktes wurde zur Messung der DNA-Konzentration nach *Burton* (Biochem. J.62, 315-323 (1956)) verwendet.

Man erhält also als Maß für die DNA-Syntese die spezifische Aktivität in Zgm/µg DNA/g Leber.

Die mit NL-Extrakt injizierten normalen Ratten zeigten unter den genannten Versuchsbedingungen im Durchschnitt eine mittlere spezifische Aktivität von 3 340 ± 1 320 Zpm/µg DNA/g Leber (Anzahl der Versuchstiere : n = 8). Bei den mit physiologischer Kochsalzlösung injizierten normalen Ratten ergab sich ein Wert von 3 470 ± 740 (n =8). Die Aktivität war also bei beiden Gruppen von Kontrolltieren im wesentlichen gleich.

Bei den mit TL-Extrakt injizierten Tieren ergab sich im Gegensatz dazu eine sehr viel größere Aktivität, nämlich eine mittlere spezifische Aktivität von 11 240 ± 4 730. Dies ergibt gegenüber den Kontrollen (NL-Extrakt von NaCl) eine Steigerung um den Faktor 3,3, und zwar mit hoher statistischer Sicherheit (p 0,01). Damit ist nachgewiesen, daß die i.p.-Injektion des in der beschriebenen Weise aus teilhepatektomierten Lebern gewonnenen Extraktes bei normalen Tieren zu einer erheblichen Steigerung der DNA-Synthese führt, die ihrerseits eine notwendige Voraussetzung einer Zellteilung und damit eines Leberwachstums durch Zellteilung ist.

Um festzustellen, ob die erfolgte Steigerung der DNA-Synthese auch tatsächlich zu einer entsprechenden wirklichen Erhöhung der Anzahl der Leberzellen führt, wurde gleichzeitig die Proliferationsrate der Leberzellen gemessen, d.h. eine Mitosezählung durchgeführt. Hierzu wurden 24 Stunden nach der Injektion von NL-bzw. TL-Extrakt die Ratten entblutet und die Leber entnommen ; vor der Entfernung wurden sie von der V. portae mit 0,9 %-iger NaCl-Lösung gespült. Die histologische Präparation erfolgte nach *Pera* (Histochem. 30, 82 (1972)) und nach *Silz et al.* (Acta Hepagastroenterol. 23, 255-261 (1976)). Hierzu wurde ein Teil des Schwanzlappens (Lobus caudatus) verwendet. Es wurden 5 μm-Schnitte durchgeführt und mit Hämatoxylin-Eosin angefärbt.

Die Mitoserate wurde durch Auszählen von 10 000 Zellen/Schnitt bestimmt.

Die Zählung der Mitoserate ergab, daß die Injektion von TL-Extrakt auch zu einer wirklichen Erhöhung der Proliferationsrate der Leberzellen geführt hatte. Der Mitoseindex betrug bei einer Doppebestimmung 7 bzw. 4 Mitosen pro $10^4$ Zellen gegenüber 0 bis 2 Mitosen pro $10^4$ Zellen bei normalen Ratten.

Dieses Ergebnis bestätigt, daß der auf die beschriebene Weise gewonnene Extrakt teilhepatektomierter Leberzellen bei einer i.p.-Injektion bei normalen Ratten zu einer echten Steigerung der Proliferationsrate der Leberzellen führt.

Zur qualitativen Bestimmung des in dem TL-Extrakt enthaltenen Faktors wurden folgende Versuche durchgeführt :

a) Enzymatische Behandlung

aa) Es wurde eine *Trypsin-Chymotrypsin*-Behandlung durchgeführt. Dazu wurden die lyophilierten wüberstände in 20 ml aqua bidest. gelöst, auf pH 7,6 gebracht und bei 30 °C für die Dauer von 2 Stunden jeweils mit 80 U Trypsin reinst und 90 U α-Chymotrypsin reinst (Serva, Heidelberg) inkubiert. Anschließend wurde für die Dauer von 30 Minuten bei 95 °C inkubiert und danach zentrifugiert. Die Lösung wurde lyophilisiert. Versuchstieren (Ratten) wurden 2 ml einer 0,9 %-igen NaCl-Lösung i.p. injiziert. Diese Behandlung führt typischerweise, sofern sie bei Proteinen oder Proteiden erfolgt, zu deren Hydrolyse und damit zu ihrer Inaktivierung.

Das ergab sich auch hier : Die Enzyminkubation führte zu Zerstörung der Aktivität. Es ergab sich nämlich eine Radioaktivität von 3 234 ± 1340 (n =5). Daraus kann gefolgert werden, daß es sich bei dem Faktor um ein Protein oder ein Proteid handelt.

bb) Ferner wurde eine *Neuraminidase*-Behandlung durchgeführt. Hierzu werden die lyophilisierten Überstände jeweils in 20 ml aqua bidest. gelöst, auf pH 5,5 gebracht und eine Stunde lang bei 37 °C mit 250 U Neuraminidase-Ansatz (Behring-Werke, Marburg) inkubiert. Anschließend wurde die überschüssige Neuraminidase bei 95 °C während 30 Minuten inaktiviert,

zentrifugiert (15 Minuten, 3 000 x g) und wieder lyophilisiert. Das Lyophilisat wurde in 2 ml 0,9 %-iger NaCl-Lösung aufgenommen und den Versuchstieren (Ratten) i.p. injiziert. Eine derartige enzymatische Behandlung führt, sofern sie an neuraminsäurehaltigen Glykoproteinen vorgenommen wird, typischerweise zur Abspaltung der Neuraminsäure und damit zu einer Inaktivierung. Eine derartige Inaktivierung erfolgte im vorliegenden Fall nicht. Es stellte sich vielmehr heraus, daß die Einbauaktivität des $^3$H-Methylthymidins in DNA und die dadurch entstandene Radioaktivität der extrahierten DNA nach wie vor sehr groß war. Es ergab sich ein Wert von 13 900 ± 5 650 (n =3). Daraus folgt, daß es sich bei dem in der oben beschriebenen Art und Weise durch Extraktion aus teilhepatektomierten Lebern gewonnenen Faktors nicht um eine Substanz handelt, die eine für die Aktivität bedeutsame Neuraminsäure besitzt.

b) Bestimmung des Molekulargewichtes

Hierzu wurde der TL-Extrakt durch ein Molekularfilter (Amicon, Lexington, USA) verschiedener Porenweite druckfiltriert. Bei der Filtration durch die Filter PM 30 wurde die gesamte Aktivität zurückgehalten. Die spezifische Aktivität des Extrakts nach Druckfiltration durch ein PM 30-Filter zeigte gegenüber dem Ausgangsmaterial eine etwa 100-fache Anreicherung. Das Molekulargewicht liegt demnach oberhalb etwa 30 000 Dalton (D). Bei Druckfiltrationen durch XM 50-Filter bleibt etwa die Hälfte der Aktivitäten im Filtrat, die andere Hälfte wurde zurückbehalten. Dies spricht dafür, daß das Molekulargewicht etwa zwischen 30 000 und 50 000 D liegt. Während die Obergrenze des Bereiches mit relativ hoher Sicherheit 50 000 D sein dürfte, ist die Untergrenze nur ein ungefährer Wert. Molekulargewichte von 20 000 und 25 000 D dürften evtl. auch noch in Frage kommen.

Die Aktivität des gefundenen Faktors ist mindestens zum organspezifisch. Der radioaktive Einbau in Milz- oder Nierengewebe war bei Injektion vo TL-Extrakt von den Kontrollen (NL bzw. NaCl) nicht unterscheidbar.

Andererseits ergab sich, daß der Faktor nicht speziesspezifisch ist. Eine Injektion des TL-Extrakts in NMRI-Mäuse (21-24 g, SPF, Institut für Strahlen- und Umweltforschung, Neuherberg/München) führte zu demselben Ergebnis wie bei Ratten (TL : 12 050 ± 6 510 ; NL: 4 350 ± 530 ; NaCl 3 240 ± 1 810 ; n = 5).

Ein *zweiter* Weg zur Gewinnung des Leberzellenproliferations-aktivierenden Faktors wird im folgenden beschrieben :

Ausgangspunkt war folgende Überlegung : Bei den beschriebenen Versuchen erfolgte die Injektion in die Bauchhöhle (i.p.) ; der TL-Extrakt muß also über den Blutkreislauf in die Leber transportiert werden. Er dürfte also auch im Blutkreislauf vorhanden sein. Deshalb wurde bei Ratten, deren Leber in derselben Art und Weise, wie oben beschrieben, teilhepatektomiert worden war, unter denselben Versuchsbedingungen das Blutplasma entnommen, und aus diesem in derselben Art und Weise, wie oben für die Restlebern beschrieben, ein Extrakt hergestellt.

Wird dieser Blutplasmaextrakt von Tieren mit teilhepatektomierter Leber (PT) normalen Ratten injiziert, so zeigt sich keine Stimulation der Proliferationsrate der Leberzellen (3 800 ± 960 ; n = 5).

Behandelt man aber einen solchen PT-Extrakt mit Neuraminidase, so ergibt sich eine hochgradig proliferationsaktive Substanz (15 380 ± 4 730 ; n =4). Damit ist ein weiterer Weg zur Gewinnung des Faktors angegeben. Ein entsprechender Extrakt aus Normalplasma erfährt nach Neuraminidasebehandlung keine Steigerung.

Wird der derart aus dem Blutplasma gewonnene Vorfaktor mit einem Trypsin-Chymotrypsin-Gemisch inkubiert, wie dies eben für den Extrakt teilhepatektomierter Lebern beschrieben wurde, so wird auch hier dadurch die Leberzellenproliferationsaktivität zerstört. Damit ist erwiesen, daß es sich bei dem aktiven Faktor aus PT ebenfalls um ein Protein oder Proteid handelt. Die Faktoren aus Leber und Plasmen sind also miteinander nahe verwandt.

Über den Wirkungsmechanismus ergibt sich daraus folgendes : Der Faktor aus TL enthält selbst keine Neuraminsäure. Der Vorfaktor im Blutkreislauf liegt dagegen in zunächst noch inaktiver Vorform als neuraminsäurehaltiges Protein vor. Durch Neuraminidase wird die Neuraminsäure abgespalten. Es entsteht ein aktiver Faktor. Nun weiß man bereits, daß bei einer Teilhepatektomie in der Leber selbst eine spontane Anreicherung der Neuraminidase erfolgt. Diese setzt dann wahrscheinlich aus dem Vorfaktor den aktiven Faktor frei. Dies erklärt auch, daß die Injektion von PT-Extrakt, also der noch inaktiven Form an sich, bei normalen Ratten noch nicht zur Erhöhung der Proliferationsrate der Leberzellen führt. Die zur Umwandlung der Vorform in das aktive Prinzip erforderliche Neuraminidase ist bei normalen, nicht teilhepatektomierten Lebern nicht bzw. nicht ausreichend vorhanden, um die Abspaltung der Neuraminsäuren und damit die endgültige Aufbereitung eines aktiven Faktors herbeizuführen. Das erfolgt erst durch die Neuraminidase, die bei hepatektomierten Lebern vermehrt vorliegt. Es ist jedoch auch möglich, daß die Umwandlung des noch inaktiven Vorfaktors in den aktiven Faktor durch Neuraminyltransferase erfolgt. Dies konnte sowohl im Blutplasma als auch in den Leberzellen selbst passieren. Daß es im Blutplasma selbst geschieht, ist allerdings wahrscheinlicher, da ja der Vorfaktor nach Behandlung mit Neuraminidase intraperitoneal injiziert wird und von dort über das Blut in die Leberzellen gelangt. Es müssen also in den Leberzellen Rezeptoren da sein, die die Neuraminsäure-freie Form binden. Dies spricht dafür, daß der Vorfaktor schon im Blut in die Neuraminsäurefreie Form übergeführt wird.

Andererseits steigt bei teilhepatektomierten Leberzellen die Neuraminidasekonzentration in der Leber stark an. Dieses dort befindliche Enzym könnte deshalb für die Umwandlung des Vorfaktors in die Neuraminsäure-freie Form ebenfalls verantwortlich sein.

Ein *dritter* bzw. *vierter* Weg zur Gewinnung des Leberzellenproliferationsaktiven Faktors besteht darin, die (nicht teilhepatektomierte vollständige) Leber bzw. das Blutplasma von foetalen Säugern oder Säugern im Alter bis zu wenigen Wochen nach der Geburt in der beschriebenen Art zu extrahieren, da man — wie eingangs erwähnt — weiß, daß bis zu diesem Alter auch bei normalen (d.h. nicht teilhepatektomierten) Tieren die Zellproliferationsrate sehr viel höher als bei älteren Tieren ist. Auch hierbei gewinnt man durch Extraktion des Blutplasmas zunächst den noch inaktiven Vorfaktor, der durch Einwirkung von Neuraminidase aktiv gemacht werden kann.

Die beschriebenen Extraktionen führen zu einer Isolation bzw. Konzentration des in den Leberzellen enthaltenen Faktors. Er ist also im Prinzip bereits im noch nicht extrahierten Leberhomogenat enthalten. Im Gesamtleberextrakt kann man ihn aber deshalb nicht nachweisen, weil solche Gesamtleberextrakte sehr giftig sind und Tiere nach Injektion regelmäßig sterben.

Die angegebenen Zeitwerte, so z.B. für den Abstand der Teilhepatektomie und Tötung der Versuchstiere, Injektion der Extrekte und Injektion des Thymidins etc. sind Werte, die sich für die Ausbeute und Reproduzierbarkeit als besonders günstig erwiesen haben. Die für die pH-Werte und die Temperatur bei der Denaturierung angegebenen Werte sind obere Grenzwerte. Der erfindungsgemäße Faktor bzw. Vorfaktor ist *gleichzeitig* bei den gennanten Werten für pH *und* Temperatur stabil.

**Ansprüche**

1. Faktor zur Stimulation der Proliferationsrate von Leberzellen, dadurch gekennzeichnet, daß er durch ein neuraminsäurefreies Protein oder Proteid mit einem Moekulargewicht von etwa 30.000 bis 50.000 D gebildet wird, das man dadurch erhält, daß von teilhepatektomierten Versuchstieren die Restlebern homogenisiert, auf einen pH-Wert von 5,5 gebracht, bei 95 °C hitzedenaturiert und zentrifugiert werden, wobei der Überstand den Faktor enthält.

2. Faktor nach Anspruch 1, dadurch gekennzeichnet, daß anstelle der Restlebern teilhepatektomierter Versuchstiere das Blutplasma teilhepatektomierter Versuchstiere eingesetzt wird, und der Extrakt mit Neuraminidase Neuraminsäure-frei gemacht worden ist.

3. Faktor nach Anspruch 1, dadurch gekennzeichnet, daß anstelle der Restlebern teilhepatektomierter Versuchstiere die nicht hepatektomierten Lebern fötaler Versuchstiere oder solcher mit einem Alter von nur wenigen Wochen eingesetzt werden.

4. Faktor nach Anspruch 1, dadurch gekennzeichnet, daß anstelle der Restlebern teilhepatektomierter Versuchstiere das Blutplasma nicht teilhepatektomierter fötaler Versuchstiere oder solcher mit einem Alter von nur wenigen Wochen eingesetzt wird, und der Extrakt mit Neuraminidase Neuraminsäure-frei gemacht worden ist.

5. Arzneimittel zur Stimulation der Proliferation von Leberzellen, enthaltend als Wirkstoff den nach einem der Ansprüche 1-4 hergestellten Leberwachstumsfaktor.

### Claims

1. Factor for stimulating the rate of proliferation of liver cells, characterized that it is a neuraminic acid-free protein or proteide having a molecular weight of approximately 30.000 to 50.000 D, which is obtained by homogenizing the remaining livers of partially hepatectomized test animals, effecting a pH-value of 5.5, heat denaturing at 95 °C and centrifuging, whereby the supernatant contains the factor.

2. Factor in accordance to claim 1, characterized in that the blood plasma of partially hepatectomized test animals is used instead of the remaining livers of partially hepatectomized test animals and that the extract is made neuraminic acid-free by neuraminidase.

3. Factor in accordance to claim 1, characterized in that the not-hepatectomized livers of foetal animals or animals with an age of only a few weeks is used instead of the remaining livers of partially hepatectomized test animals.

4. Factor in accordance to claim 1, characterized in that blood plasma of not-partially hepatectomized foetal test animals or animals with an age of only a few weeks is used instead of the remaining livers of partially hepatectomized test animals and that the extract is made neuraminic acid-free with neuraminidase.

5. Pharmaceutical drug for the stimulation of the proliferation of liver cells, containing as effective ingredient the liver growth factor produced in accordance to one of the claims 1 to 4.

### Revendications

1. Facteur de stimulation de la vitesse de prolifération des cellules hépatiques, caractérisé en ce qu'il est formé par une protéine ou un protéide ne contenant pas de neuraminacide, ayant un poids moléculaire d'environ 30 000 à 50 000 D, que l'on obtient en homogénéisant les restes de foie d'animaux d'expérience hépatectomisés, en les portant à un pH de 5,5, en les dénaturant par la chaleur à 95 °C et en les centrifugeant, le surnageant contenant le facteur.

2. Facteur selon la revendication 1, caractérisé en ce qu'on utilise, à la place des restes de foie d'animaux d'expérience hépatectomisés, le plasma sanguin d'animaux d'expérience hépatectomisés, et que l'on élimine le neuraminacide de l'extrait à l'aide de neuraminidase.

3. Facteur selon la revendication 1, caractérisé en ce qu'on utilise, à la place des restes de foies d'animaux d'expérience hépatectomisés, des animaux d'expérience à l'état fœtal ou âgés seulement de quelques semaines.

4. Facteur selon la revendication 1, caractérisé en ce qu'on utilise, à la place des restes de foies d'animaux d'expérience hépatectomisés, le plasma sanguin d'animaux d'expérience à l'état fœtal ou âgés seulement de quelques semaines, non hépatectomisés, et en ce qu'on élimine le neuraminacide de l'extrait à l'aide de neuraminidase.

5. Médicament servant à stimuler la prolifération des cellules hépatiques, contenant comme agent actif le facteur de croissance du foie fabriqué selon l'une quelconque des revendications 1 à 4.